# EUROPEAN PATENT APPLICATION

(11) **EP 3 913 370 A1**
(43) Date of publication of application: **24.11.2021**
(21) Application number: 20175139.3
(22) Date of filing: 18.05.2020
(51) Int. Cl.: G01N 33/574

(54) **METHOD FOR DIAGNOSING CARCINOMAS USING IRGD AS A TUMOR MARKER BOOSTER**

(71) Applicant: Piiper, Albrecht, 69121 Heidelberg (DE)
(72) Inventor: PIIPER, Albrecht, 69121 Heidelberg (DE); KAKOSCHKY, Bianca, 60529 Frankfurt am Main (DE); DENK, Dominic, 60528 Frankfurt am Main (DE); SCHMITHALS, Christian, 60528 Frankfurt (DE)
(74) Representative: Simandi, Claus

(57) **Abstract**

The present invention relates to a method for the diagnosis and/or risk stratification of carcinoma diseases of patients and subjects using iRGD and a related kit.

## Description

The present invention relates to a method for the diagnosis and/or risk stratification of carcinoma diseases of patients and subjects using iRGD and a related kit.

For the majority of cancers, early detection enables the application of potentially curative treatments, leading to improved patient survival, for instance, in liver cancer, the third leading cause of cancer-related mortality worldwide, among which hepatocellular carcinoma (HCC) accounts for the majority of cases. The 5-year survival rate is less than 10% for advanced-stage HCC, but 50-80% for early-stage patients (1-3) .

Curative treatment approaches of HCC are liver transplant or resection. While a transplant can be carried out in BCLC stages O, A, and possibly B, tumor resection is only useful in stage 0. The remaining, more advanced tumor stages can solely be treated by local ablative procedures (LITT, TACE) or palliatively by systemic treatment (chemotherapy with sorafenib). As a result, making an early diagnosis is crucial for a successful treatment of solid malignant neoplasms.

Improved monitoring of patients at high risk of developing tumors or neoplasms, which is to say patients suffering from liver cirrhosis, and diagnosing suspicious nodules are of central importance for a considerable improvement in the prognosis of cancer patients.

Small HCCs, however, rarely exhibit the typical radiological criteria and are rather hypovascular because the supply via the portal vein is already reduced, and because arterial hypervascularization has not yet formed. Consequently, it remains difficult to make the clinically extremely significant distinction between small HCCs and regenerate and dysplastic nodules in the cirrhotic liver.

Unfortunately, due to a lack of methods for early diagnosis, most cancer patients are diagnosed at an advanced stage with limited therapeutic options and thus poor prognosis. Tumor markers that detect malignant tumors at an early stage with high sensitivity and specificity could improve this situation. However, the tumor markers currently in clinical use, such as α-fetoprotein (AFP) for HCC and prostate-specific antigen (PSA) for prostate cancer (4-6) generally show insufficient sensitivity and specificity to be effective in early diagnosis of cancer. Elevated blood levels associated with non-malignant conditions (e. g. inflammation), variable tumor expression, and dilution of small amounts released by small (early) tumors in the large blood volume are the main reasons for false positive and negative results (7).

Recent publications (8-10) disclose a new RGD peptide, so called iRGD, possessing remarkable tumor-internalizing and tumor-penetrating properties. Within minutes after its intravenous injection in a subject, iRGD triggers transport of substances coupled or linked to iRGD or co-administered with it from the blood into tumors. The iRGD-augmented transport is specific for tumors and leads to a severalfold increase of the level of co-administered therapeutics in malignant tissue, both primary tumors and metastases but not in normal tissues. The result is a corresponding increase of the therapeutic index of the co-administered substance. The effect of iRGD has been documented in many mouse tumor models, and with a variety of drugs, indicating broad applicability (10,11).

Furthermore, WO 2011/005540 A1 describes the therapeutic use by way of iRGD for the improved penetration of cancer drugs into cancer cells.

iRGD binds to αvβ3/5 integrins, which are specifically expressed on tumor vessels, thereby conferring tumor specificity to the iRGD effect. The integrin binding is followed by proteolytic cleavage of the peptide, resulting in the generation of a truncated peptide with a neuropilin-1 (NRP-1)-binding motif (CendR motif). This CendR peptide then binds to NRP-1 on tumor vessels and tumor cells and thereby activates an endocytotic vesicular trans-tissue transport pathway (8,12). The iRGD-induced trans-endothelial transport has been reported to involve the formation of grape-like vesicles spread across the endothelial cells (13) resembling the vesiculo-vacuolar organelle described by Dvorak and coworkers (14) and/or opening of endothelial cell-cell contacts (15). Pre-clinical studies have indicated that there is no toxicity associated with iRGD administration and that iRGD inhibits metastasis (16). The first-in-man study with iRGD currently examines the tolerability and ability of iRGD to improve drug therapy in patients with pancreatic carcinoma (NCT03517176) and shows low toxicity of iRGD in humans.

Proceeding from this prior art, it is therefore the object of the invention to provide new options for the diagnosis, differential diagnosis, prognosis, and in particular for the early detection, of carcinomas, in particular of hepatocellular carcinoma (HCC), and for the risk stratification of such carcinoma diseases.

Now, the inventors surprisingly have found that iRGD also affects tumor-to-blood transport providing an increased release of tumor specific markers or tumor dependent markers into the bodily fluid, particularly blood, which significantly improve the specificity of released tumor markers in the blood or bodily fluids of a subject.

The above-described object is surprisingly achieved by the use of iRGD as a tumor specific marker or tumor dependent marker enhancer or booster in a method for the diagnosis and/or risk stratification of carcinoma diseases or carcinomas.

Advantageously, iRGD allows the increased release of tumor markers from a tumor resulting in a higher amount of tumor markers for their detection resulting in an improved specificity of released tumor markers in the blood or bodily fluids of a subject.

The results as indicated in the examples and figures suggest a new property for the CendR pathway, namely the transport of substances in the opposite direction, and accordingly from the tumor to the blood. The existence of this reverse transport was indicated by the appearance of increased tumor marker protein levels like alpha fetoprotein (AFP) or prostate-specific antigen (PSA) (cf. examples) and of the secretory protein autotaxin (ATX) in the circulation as a result of iRGD administration to mice with carcimoma, like HCC and prostate cancer as provided in the examples.

No such effect was observed in mice with chronic liver injury/fibrosis without HCC, although some of these mice showed elevated basal blood AFP levels.

Thus, the iRGD-induced tumor export is specific for malignancy, similar to the tumor-specific effect of iRGD on the delivery of substances into tumors (9,10). The apparent specificity of the iRGD-induced reverse transport in tumors provides an advantageous approach to overcome the difficulty of determining whether an increase of a circulating marker originates from a tumor or from a non-malignant tissues that produces the marker, such AFP in liver cancer vs. hepatitis.

The application of iRGD increases the specificity for tumors of a protein lacking tumor specificity per se, such as ATX.

The accuracy of AFP to indicate the presence of HCC is particularly low in patients with small early stage HCCs, which produce AFP in amounts too small to elevate AFP level in the blood (18,23). The inventors have found that iRGD increased the blood AFP level in a large portion of TGFa/c-myc mice with small to medium-sized HCC tumors and basally normal AFP levels. Thus, the additional transport of AFP into the blood elicited by iRGD increases the sensitivity of the AFP test in early-stage HCC.

The putative iRGD-induced tumor-to-blood transport may occur through the endocytic vesicular trans-tissue transport pathway suggested for iRGD-induced tumor uptake of drugs (12,13), as iRGD-induced tumor release of AFP depends on NRP-1 similar to iRGD-induced tumor uptake, and the timing of the two effects is similar. The direction of the sum transport may be determined by the concentration gradient between the blood and tumor. AFP, ATX and PSA share the characteristic that they are secreted by the constitutive secretory pathway.

Our finding that iRGD also causes an increase of the blood levels of PSA in a mouse model of prostate cancer indicates that the iRGD-induced tumor-to-blood transport extends to at least one tumor marker and cancer type other than AFP. HCC may be broadly representative of malignant tumors, similar to the broad presence of iRGD-induced tumor import in different tumor types (10). Given the broad use of PSA in prostate cancer diagnosis, it is important to note that iRGD provides a way of improving the accuracy of this test.

Systemic administration of iRGD to patients might also be informative regarding responsiveness of a patient to iRGD-augmented tumor therapy. Data from patient-derived pancreatic cancer xenografted mice indicate that differential expression of NRP-1 in the tumor vessels influences the efficacy of iRGD in promoting tumor influx (13). Thus, measurement of tumor specific markers or tumor dependent markers and their levels before and after the application of iRGD is a method according to the invention to diagnose or stratify the patients or subjects with respect to their iRGD responsiveness.

The invention thus relates to a method for the diagnosis and/or risk stratification of carcinoma diseases, by the following steps
a.) iRGD is applied to a subject,
b.) a sample of a bodily fluid is provided from said subject,
c.) determining or identifying in said sample one or more marker released from a tumor into the bodily fluid.

In a preferred embodiment of the invention, in step c.) the concentration or level of said one or more marker derived from said tumor is determined and in a further step d.) correlating the determined level to a potential risk for carcinoma.

The term "one or more marker released from a tumor into the bodily fluid" according to the invention refers to any chemical substances, preferably proteins, peptides, which are detectable or identifiable in the blood, in particular in the bloodstream, or other bodily fluids of a tumor patient or subject, wherein i.) an altered, particularly increased concentration or level indicates a tumor or a tumor activity, like an adverse event or outcome, or wherein ii.) an identified marker indicates the presence of a tumor.

In a preferred embodiment of the invention, step c.) correlates to i.) an altered, particularly increased concentration or level of a marker indicating a tumor or a tumor activity, like an adverse event or outcome, or ii.) an identified marker indicating the presence of a tumor.

In a preferred embodiment of the invention, at least one marker is determined before and after the administration of iRGD, in particular independent from each other one week, preferably three days, 48 hours or 24 hours.

Hence, in a further preferred embodiment according to the invention, the invention relates to a method for the diagnosis and/or risk stratification of carcinoma diseases, by the following steps
a'.) a first sample of a bodily fluid is provided from said subject,
a.) iRGD is applied to a subject,
b.) a second sample of a bodily fluid is provided from said subject,
c.) determining or identifying in said sample one or more marker released from a tumor into the bodily fluid and
c'.) determining the difference of the marker levels between the first sample a'.) and second sample b.).

Hence, the present invention encompasses such "one or more marker released from a tumor into the bodily fluid" selected from the group of tumor markers, like circulating tumor markers, tumor tissue markers and any tumor specific or tumor dependent markers, which are released by the tumor into the blood or bloodstream or bodily fluid due to their iRGD responsiveness after application of iRGD.

The majority of such tumor markers is preferably selected from the group, but not limited to, of carcinoembryonic antigen (CEA), alpha fetoprotein (AFP), carbohydrate antigen 19/9 (CA 19-9), cancer antigen 72/4 (CA 72-4), cancer antigen 125, cancer antigen 15/3 (CA 15-3), neuron-specific enolase (NSE), squamous cell carcinoma antigen (SCC), cytokeratin fragment (CYFRA), human chorionic gonadotropin (HCG), prostate-specific antigen (PSA), human thyroglobulin (HTG), mucin-like cancer associated antigen (MCA), nuclear matrix protein 22 (NMP22), human placental alkaline phosphatase (hPALP), calcitonin, myopodin, prostate-specific membrane antigen (PSMA), pyruvate kinase M2 (PKM2), S100 proteins, thymidine kinase (TK) and tissue polypeptide antigen (TPA), which are produced and released into the bodily fluid, particularly blood by the tumor cells.

The term "any tumor specific or tumor dependent markers" (supra) refers to any released chemical substances due to iRGD responsiveness after application of iRGD such as autotaxin (cf. examples), a secreted protein highly expressed in inflamed tissue and tumors (35), which can be detected in bodily fluid, particularly blood. Such released chemical substances are preferably selected from proteins, peptides and others.

According to the invention, preferred carcinomas are those that are related to a disturbance of the barrier function of the vessels and decreased lymphatic drainage in the region subject to pathological changes. This applies to solid malignant neoplasms, breast cancer, colon cancer, lung cancer, pancreatic cancer, stomach cancer, ovarian cancer, biliary duct cancer, prostate cancer, cervical cancer, glioblastoma, bronchial cancer, renal cell cancer, bladder cancer, sarcomas, liver cancer, hepatocellular carcinoma (HCC), brain tumors, seminal vesicle tumors, thyroid cancer, medullary thyroid cancer, melanoma, leukemia, lymphoma or tumor metastases of these cancers.

In one particularly preferred embodiment, the said tumor specific or tumor dependent marker enhancer or booster according to the invention furthermore comprises a tumor-homing peptide, and in particular iRGD, preferably having the sequences CRGDKGPDC (SEQ ID No.1), CRGDRGPDC (SEQ ID No.2), CRGDKGPEC (SEQ ID No.3) or CRGDRGPEC (SEQ ID No.4).

The administration of iRGD according to the invention, can be carried out in any manner best known to a person skilled in the art. This includes in particular the intravenous, parenteral, oral, rectal administration, and so forth, of different formulations of iRGD. The required dose is varied as a function of the age, the size and the weight of the patient.

iRGD may be used alone or in combination with other diagnostic, therapeutic or other substances. "Other substances" shall in particular be understood to mean pharmaceutical adjuvants, flavor additives or dyes. For example, sucrose or a natural citrus flavor may be admixed to an orally administered formulation.

In a preferred embodiment said iRGD is applied, injected or administered into the bloodstream of a subject via a hollow hypodermic needle and a syringe or infusion at higher pressure, which is pierced through the skin into the body, so called intravenous application, comprising intramuscular or subcutaneous application. Hence, in a preferred embodiment of the invention iRGD is administered via intravenous, intramuscular or subcutaneous application in a subject.

Hence, the present invention refers to the use of iRGD as a tumor specific or tumor dependent marker enhancer or booster in a method for the diagnosis and/or risk stratification of carcinoma diseases or carcinomas.

The term "correlating" as used herein in reference to the use of the tumor specific or tumor dependent markers according to the invention, refers to correlating the presence or amount, like concentration or level of the released marker in the bodily fluid of a patient to its presence or amount, like concentration or level in persons known to suffer from, or known to be at risk of, a given condition. A marker level in a patient sample can be compared or correlated to a level known to be associated with a specific prognosis. The sample's marker level is said to have been correlated with a prognosis; that is, the skilled artisan can use the marker level to determine whether the patient has a specific risk to suffer from an adverse event, and respond accordingly. Alternatively, the sample's marker level can be compared or correlated to a marker level known to be associated with a good outcome (e.g., a low risk to suffer from an adverse event).

The term "sample" as used herein refers to a sample of bodily fluid obtained for the purpose of assessment, diagnosis, prognosis, or evaluation of a subject of interest, treatment guidance such as a patient. Preferred test samples include blood, serum, plasma, cerebrospinal fluid, urine, saliva, sputum, and pleural effusions. In addition, one skilled in the art would realize that some test samples would be more readily analyzed following a fractionation or purification procedure, for example, separation of whole blood into serum or plasma components.

Thus, in a preferred embodiment of the invention the sample is selected from the group comprising a blood sample, a serum sample, a plasma sample, a cerebrospinal fluid sample, a saliva sample and a urine sample or an extract of any of the aforementioned samples. Preferably, the sample is a blood sample, most preferably a serum sample or a plasma sample.

The term "plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g., citrated, EDTA or heparinized blood), for example for at least 15 minutes at 2000 to 3000 g.

The term "serum" in the context of the present invention is the liquid fraction of whole blood that is collected after the blood is allowed to clot. When coagulated blood (clotted blood) is centrifuged serum can be obtained as supernatant.

The term "diagnosis" in the context of the present invention relates to the recognition and detection of a carcinoma in a subject and/ or the ruling in or out of a carcinoma and the related severity. The diagnostic method according to the invention is performed *ex vivo*/*in vitro,* in particular by obtaining a bodily fluid.

The term "carcinoma or cancer / cancerous conditions" summaries a class of diseases which have the common factor that they form malignant tumors. To date, more than 200 different tumors have been identified. What is characteristic for all malignant tumors is the uncontrolled proliferation of cells, the ability to displace healthy tissue (invasion of the adjacent tissue), and the ability to form metastases in the tissue of the entire body (distant metastases). These three processes are characteristic for the progression of cancer and are used as criteria for classifying cancer into cancer stages I, II, III and IV (or A-D) and for staging by TNM classification and determining the aggressiveness of the disease. From stage III (C), it is possible to determine that the tumor is outgrowing its area of original formation and is displacing the surrounding tissue. From stage IV (D), distant metastases can be determined. Depending on the stage of cancer, different treatment is recommended so as to achieve a successful result. Early identification and assessment of the aggressiveness of the tumor cells are therefore of great importance in order to select a suitable course of therapy for a patient. Cancerous conditions are diagnosed in particular by imaging methods, such as ultrasound, MRI, CT and the like, and by the detection of tumor specific or tumor dependent markers.

The preferred detection methods for marker(s) derived from tumors according to the invention comprise means like immunoassays in various formats such as for instance luminescence immunoassay (LIA), radioimmunoassay (RIA), chemiluminescence- and fluorescence- immunoassays, enzyme immunoassay (EIA), Enzyme-linked immunoassays (ELISA), luminescence-based bead arrays, magnetic beads based arrays, protein microarray assays, rapid test formats such as for instance immunochromatographic strip tests, rare cryptate assay, and automated systems/analyzers.

The assays can be homogenous or heterogeneous assays, competitive and non-competitive assays. In a particularly preferred embodiment, the assay is in the form of a sandwich assay, which is a non-competitive immunoassay, wherein the molecule to be detected and/or quantified is bound to a first antibody and to a second antibody. The first antibody may be bound to a solid phase, e.g., a bead, a surface of a well or other container, a chip or a strip, and the second antibody is an antibody which is labeled, e.g., with a dye, with a radioisotope, or a reactive or catalytically active moiety. The amount of labeled antibody bound to the analyte is then measured by an appropriate method. The general composition and procedures involved with "sandwich assays" are well-established and known to the skilled person.

In a preferred embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, auch as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M-1.

In a further embodiment, the invention relates to a kit and use thereof, comprising one or more injection solutions for carrying out a method according to the invention, comprising iRGD and means for detecting at least one tumor marker as outlined above.

For example, an injection solution may comprise about 300 µmol iRGD for a patient weighing 75 kg (4 µmol/kg).

According to the invention, the term "risk stratification" comprises the identification of patients, in particular at-risk patients, having a worse prognosis, for the purpose of more in-depth diagnostics and therapy/treatment of carcinoma diseases with the goal of enabling as favorable a progression of the disease as possible. Risk stratification according to the invention consequently allows effective treatment processes, which in the case of carcinoma diseases are achieved through newer drugs, such as cytostatic drugs, monoclonal antibodies and chemotherapy, for example, or are used for the treatment or therapy of carcinoma diseases.

The term "subject or synonymous patient" as used herein refers to a living human or non-human organism that is receiving medical care or that should receive medical care due to a disease, in particular carcinoma. This includes persons with no defined illness who are being investigated for signs of pathology. Thus, the methods described herein are applicable to both, human and veterinary disease.

The invention therefore likewise relates to the identification of patients who are at an increased risk of and/or have an unfavorable prognosis for carcinoma diseases, and more particularly in symptomatic and/or asymptomatic patients, in particular patients at risk, for example due to metastasis.

Particularly advantageously, reliable stratification of patients can take place by way of the method according to the invention. The method according to the invention thus enables clinical decisions that result in rapid treatment success and the prevention of deaths or critical outcome. Such clinical decisions likewise include advanced treatment using drugs for the treatment or therapy of carcinomas.

The invention thus likewise relates to a method for the diagnosis and/or risk stratification of patients affected by carcinoma diseases for carrying out clinical decisions, such as advanced treatment and therapy using drugs, including the decision to hospitalize the patient.

In a further preferred embodiment, the method according to the invention thus relates to the therapy control of carcinoma diseases or carcinomas.

In a further preferred embodiment of the method according to the invention, for the diagnosis and/or risk stratification takes place for prognosis, for early detection and detection by differential diagnosis, for assessment of the severity, and for assessment of the course of the disease concomitant with the therapy.

The present invention is to be described in greater detail hereafter based on examples and figures, without thereby limiting the invention.

### Examples:

### Example 1:

### Intravenously injected iRGD leads to a rapid increase in the blood AFP level specifically in mice with HCC:

We reasoned that iRGD may also induce an export of substances from tumors to the blood which should be reflected in elevated levels of tumor markers in the circulation. As iRGD induces a rapid (within minutes) activation of the CendR pathway (10), which fades rapidly due to the short circulation half-life of iRGD (17), we expected a rapid increase in the blood concentration of AFP upon iRGD administration. AFP is a protein expressed and secreted by approximately half of human HCCs, leading to elevated AFP levels in the blood of affected patients (4,5,18). The blood half-life of AFP is several days in humans. Thus, even a short iRGD-induced spike in AFP transport from the tumor would be expected to cause elevated blood AFP levels for at least several hours.

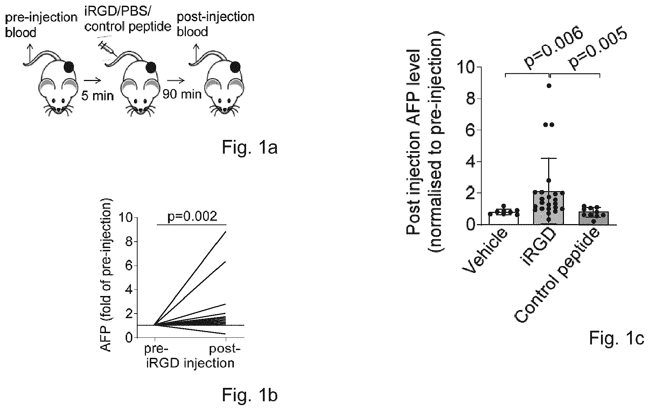

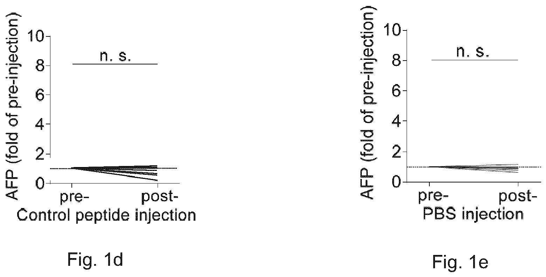

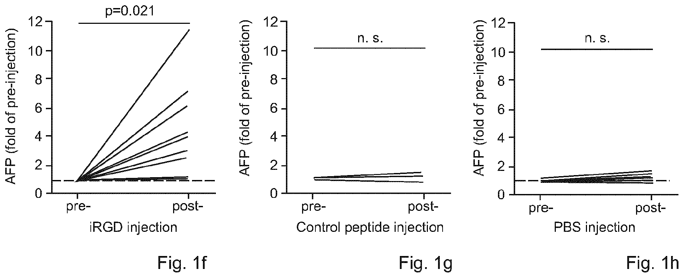

We injected nude mice bearing AFP-expressing HCC xenografts, which show iRGD-induced tumor uptake (19) with iRGD, an RGD control peptide or PBS, and analysed blood samples drawn 5 minutes before and 90 minutes after the injections for AFP concentrations (Fig. 1a). iRGD caused an increase of the blood AFP levels in mice with HepG2 (Fig. 1b-e) and Huh-7 tumors (Fig. 1f-h). The blood AFP levels were approximately two-fold higher than the levels prior to the iRGD injection (Fig. 1c). An RGD control peptide lacking the CendR motif or PBS had no effect (Fig. 1d, 1g, 1h). Thus, iRGD causes an acute increase of the blood AFP levels in mice bearing AFP-producing HCC tumors.

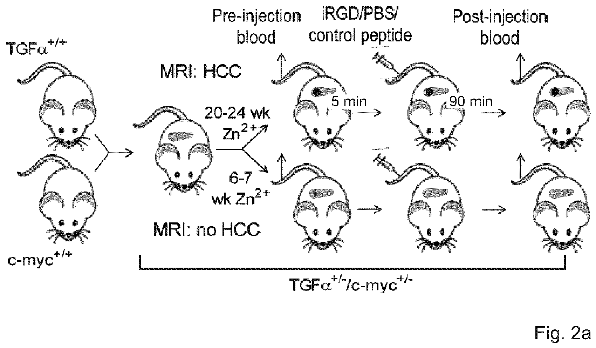

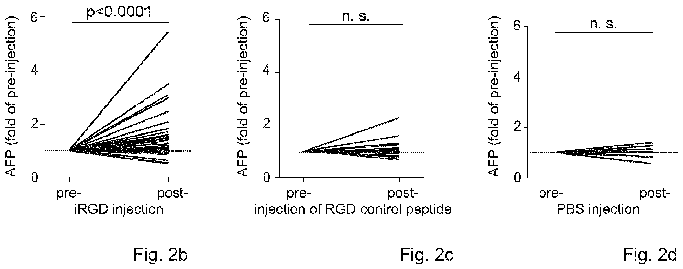

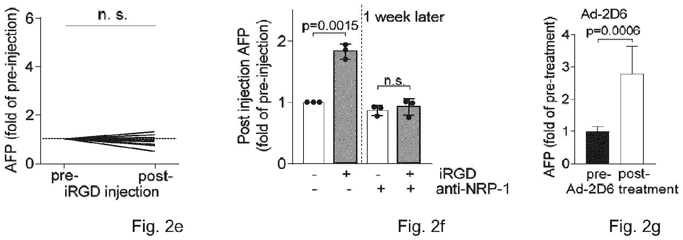

To investigate, if the iRGD-induced increase in the blood AFP level also occurs in mice with endogenously formed HCCs and can be discriminated from the background AFP produced by extra-tumoral sites, we examined the effect of iRGD on the blood AFP level in conditional double transgenic TGFa/c-myc mice with HCC. These mice develop HCCs upon induction of transgene expression by zinc in the drinking water (20,21) and iRGD causes tumor uptake of intravenously co-injected Evans blue in the tumors of these mice (16). Intravenous injection of iRGD into TGFa/c-myc mice with HCCs caused an increase in the blood AFP levels (Fig. 2a, b), whereas RGD control peptide or PBS had no effect (Fig. 2c, d); iRGD did not influence blood AFP levels of the TGFa/c-myc mice prior to tumor development at 6-7 weeks of age (Fig. 2e). Thus, iRGD increases blood AFP levels in transgenic tumor mice but only after they have developed tumors.

To investigate whether the iRGD-induced increase in the blood AFP level in HCC mice depends on NRP-1 as does iRGD-induced tumor import (8,10,12) we studied the effect of a neutralizing anti-NRP-1 antibody on iRGD-induced increase of the blood AFP level in the TGFa/c-myc HCC mice that had given a robust blood AFP response to iRGD one week earlier. The mice injected with anti-NRP-1 no longer exhibited the iRGD-induced blood AFP elevation (Fig. 2f), indicating NRP-1-dependence of the iRGD-induced increase in AFP.

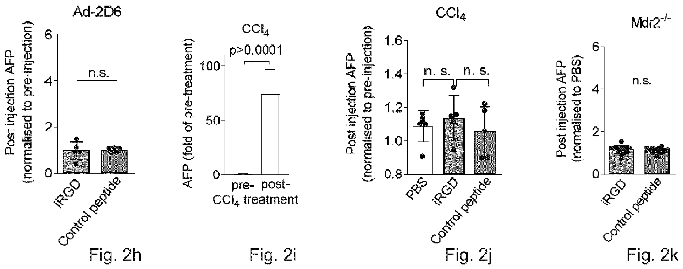

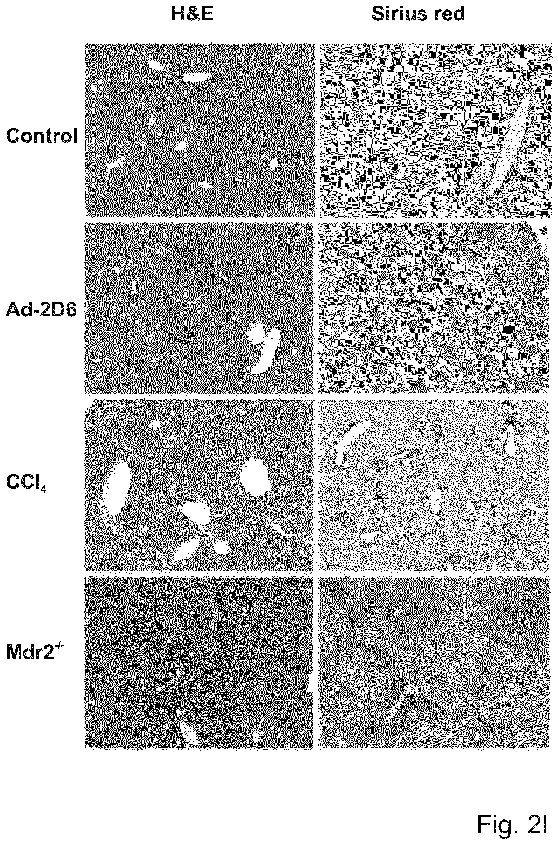

As patients with chronic inflammatory liver disease frequently show elevated blood AFP levels in the absence of a tumor (18,22,23) we investigated the effect of iRGD on blood AFP levels in three different mouse models of liver fibrosis (Ad-2D6 virus-induced autoimmune hepatitis, Mdr2-/- mice or CCl₄ treatment). The Ad-2D6 and CCl₄-treated mice with histologically confirmed liver fibrosis (Fig. 2l) presented with elevated blood AFP (Fig. 2h, i), whereas the Mdr2-/- mice had normal AFP levels. iRGD treatment did not increase blood AFP levels in any of the three models (Fig. 2h, j, k), suggesting that iRGD does not affect tissue-to-blood transport of increased AFP levels produced by non-malignant tissue. Thus, AFP in combination with iRGD is more specific for HCC than the absolute level of AFP.

### Example 2:

### iRGD-induced elevation of the blood AFP concentration occurs mainly in mice with small HCCs showing normal blood AFP without iRGD:

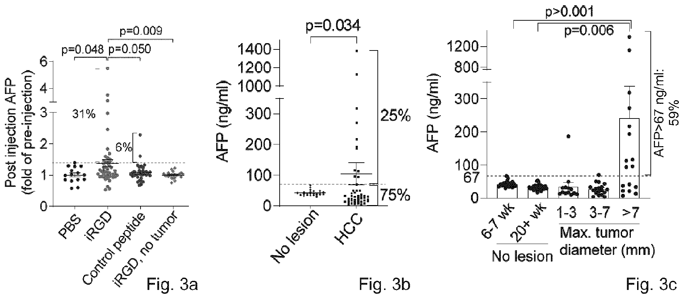

We next investigated the influence of iRGD on the sensitivity of AFP in detecting HCC. 31% of TGFa/c-myc HCC mice showed a stronger increase in the blood AFP level upon injection of iRGD than any of the PBS-injected, HCC bearing mice (Fig. 3a). Meanwhile, only 25% of the TGFa/c-myc HCC mice exhibited basally elevated blood AFP levels when compared to TGFa/c-myc mice at 6-7 months of age, i. e. prior to HCC development (Fig. 3b). Examination of the dependence of the basal blood AFP level on tumor size revealed that basal blood AFP levels were normal in mice with small or medium size HCCs and tended to be elevated in TGFa/c-myc mice with large HCCs (Fig. 3c), in agreement with the observation that patients with small HCCs often produce too small amounts of AFP to elicit an elevation of the blood AFP level (18,22,23).

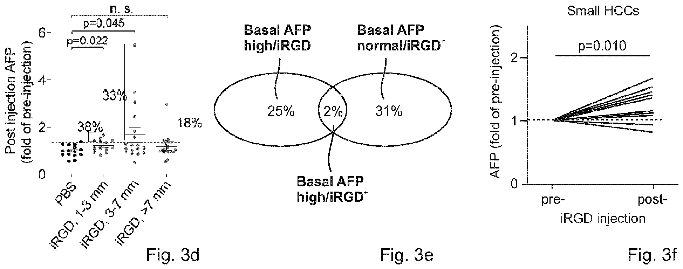

iRGD treatment elevated blood AFP in a substantial portion of mice with small and medium size liver tumors having normal blood AFP levels without iRGD (Fig. 3d, Fig. 3f-i). In contrast, iRGD had no statistically significant effect on blood AFP levels in mice with large HCCs (Fig. 3d, Fig. 3f-i). Interestingly, there was little overlap between HCC mice showing basally elevated blood AFP levels and those revealing iRGD-induced elevation of the blood AFP concentration (Fig. 3e), indicating that iRGD-induced elevation of the blood AFP level occurred mainly in HCC mice with basally normal blood AFP levels. Together, these data indicate that iRGD induces an efficient tumor-to-blood transport of AFP in mice with small (early) HCCs, leading to a strong increase of the sensitivity to detect early stage HCC.

### Example 3:

### iRGD also induces an increase of the blood level of autotaxin and of PSA in mice with prostate cancer:

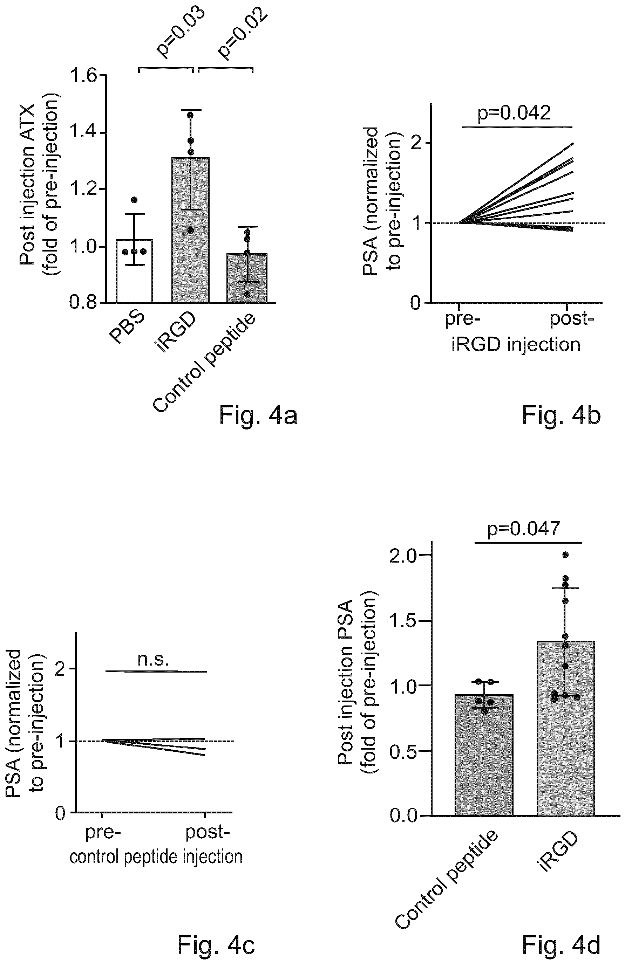

Increased transport into the blood of tumor-released proteins other than AFP by iRGD could considerably broaden the utility of iRGD in tumor detection. To address this issue, we examined the iRGD responsiveness of blood levels of autotaxin, a secreted protein highly expressed in inflamed tissue and tumors (24). iRGD caused an increase of the blood levels of autotaxin in nude mice xenografted with HepG2 tumors, whereas the RGD control peptide and vehicle had no effect (Fig. 4a). Thus, iRGD appears to increase tumor-to-blood transport of autotaxin, a protein that does not have tumor marker properties without iRGD.

To examine whether iRGD induces a tumor-to blood transport of other tumor markers in another type of tumor, we investigated the effect of iRGD on blood levels of PSA in prostate cancer mice. iRGD caused an average of 1.3-fold increase in the blood PSA level nude mice xenografted with LNCaP tumors (Fig. 4b, c). This change was not observed with the RGD control peptide (Fig. 4c, d). iRGD responsiveness of this clinically important tumor marker suggests broader applicability of the iRGD effect on different tumor markers and tumor types.

### Figures:

**Fig. 1****:** Intravenously injected iRGD increases blood AFP levels in HCC-bearing mice.
   **a:** Scheme of the experimental setup.
   **b-e:** Blood was drawn from nude mice xenografted with HepG2 tumors five min before and 90 min after an i. v. injection of iRGD (4 µmol/kg) (b (n=18), RGD control peptide (4 µmol/kg) (**d** (n=10), or vehicle (PBS) (**e** (n=9)). Serum AFP levels were determined by ELISA. c Effect of iRGD on post-treatment levels of AFP as fold of the pre-injection level. Columns and error bars represent means ± SD. Human AFP was not detectable in the blood of mice without tumors.
   **b, d, e:** Wilcoxon signed rank test; **c:** Mann-Whitney U test. Intravenously injected iRGD increases blood AFP levels in Huh-7 tumor xenograft-bearing mice. Blood was drawn from nude mice xenografted with Huh-7 tumors five min before and 90 min after an i. v. injection of iRGD (4 µmol/kg) (**f** (n=12), RGD control peptide (4 µmol/kg) (**g** (n=6), or vehicle (PBS) (**h** (n=6)). Serum AFP levels were determined by ELISA; n. s., not significant; Wilcoxon signed rank test.
**Fig. 2****:** iRGD increases blood AFP level in TGFa/c-myc mice with HCC, but not in mice with chronic liver injury/fibrosis.
   **a:** Scheme of the experimental setup.
   **b-g:** iRGD specifically increases the blood AFP levels in TGFa/c-myc HCC mice. TGFa/c-myc mice (20-24 weeks old) with HCC **(b-d)** and mice prior to HCC development (6-7 weeks of age, n=23) **(e)** were intravenously injected with iRGD (**b**, n=48), RGD control peptide (**c**, n=33), or vehicle (**d**, PBS, n=15). Blood was drawn 5 min before and 90 min after the injection. Serum AFP levels were determined by ELISA.
   **f:** TGFa/c-myc tumor mice that displayed a robust iRGD-induced increase in blood AFP level one week earlier were injected with anti-NRP-1 and the effect of iRGD on blood AFP level was determined. Columns and error bars represent means ± SD; n=3 per group. Asterisks indicate significant differences (**P<0.01); n. s., not significant.
   **g-k:** Mice with liver fibrosis do not show iRGD-induced elevation of the blood AFP level.
   **g-j:** Liver fibrosis was induced by treating mice with Ad-2D6 (g, h) or CCl₄ (**i, j**) for four weeks.
   **g-i:** Blood was drawn from the mice before the treatment and one day after the treatment was completed and was analyzed for AFP content. Five min later, iRGD, RGD control peptide or PBS were injected intravenously, and blood was drawn 2 hours later. Six-month-old Mdr2-/- mice **(k),** which develop liver fibrosis spontaneously, were bled before and after iRGD injection. All sera were examined for AFP content. Columns and error bars represent means ± SD (g, h: 5-6 per group; **i, j:** 5-6 per group; **k:** n=17 per group); n. s., not significant.
   **b-e** Wilcoxon signed rank test; **f-k** Mann-Whitney U test.
   **1:** Histological evidence of liver fibrosis in Ad-2D6 and Mdr2^{-/-} mice, and hepatitis/fibrosis induced by CCl₄. Mice were treated with Ad-2D6 or CCl₄ for four weeks. Six months-old Mdr2^{-/-} mice develop liver fibrosis spontaneously. Paraffin-embedded liver slices were stained with Hematoxylin/eosin (H&E) and Sirius red (n=5 per group).

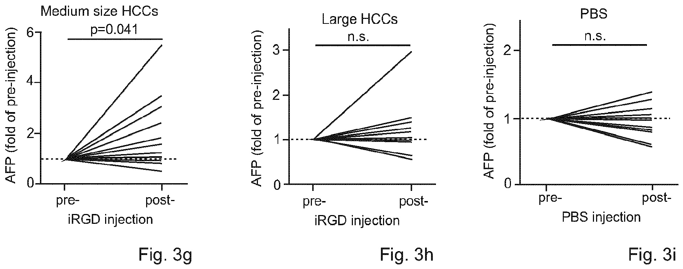

**Fig. 3****:** iRGD induces an increase of the blood AFP level in TGFa/c-myc mice with small/medium size HCCs and normal basal blood AFP levels.
   **a:** Portion of TGFa/c-myc mice with HCC showing an iRGD-induced increase in the blood AFP level in TGFa/c-myc mice (20-24 weeks old) with HCC confirmed by contrast-enhanced MRI or mice prior to HCC development (6-7 weeks of age, n=23) were intravenously injected with iRGD (n=48), RGD control peptide (n=33), or vehicle (PBS, n=15). Blood was drawn 5 min before and 90 min after i. v. injection of iRGD, RGD control peptide or PBS. Serum AFP levels were determined by ELISA. Lines and error bars indicate means ± SEM.
   **b:** AFP levels in TGFa/c-myc mice with HCC tumors confirmed by contrast-enhanced MRI (n=48) or with no tumor (n=23). The lines and error bars indicate means ± SEM.
   **c:** Tumor size-dependence of blood AFP level in TGFa/c-myc mice. AFP levels in TGFa/c-myc mice with no macroscopic liver tumors (6-7 weeks, n=23, >20 weeks, n=40) or tumors with maximal diameters of 1-3 mm (n=13), 3-7 mm (n=18) or >7 mm (n=17). Columns represent mean AFP levels ± SEM.
   **d:** The tumor size is a critical parameter for presence of iRGD-induced increase of the blood AFP level in TGFa/c-myc mice. Mice (20-27 weeks old) with small (1-3 mm maximum diameter, n=13), intermediate (3-7 mm maximum diameter, n=18) or large (>7 mm maximum diameter, n=17) liver tumors were injected with iRGD. Serum was obtained five min before and 90 min after the injections and analyzed for AFP content. Lines and error bars indicate means ± SEM.
   **a-d:** Mann-Whitney U test.
   **e:** Little overlap between TGFa/c-myc HCC mice showing basally elevated blood AFP level and those showing iRGD-induced increase in the blood AFP concentration (n=48). iRGD- = mice showing no iRGD-induced alteration of the blood AFP level, iRGD+ = mice showing an iRGD-induced increase in the blood AFP level.
   **f-i:** iRGD responsiveness of blood AFP level most pronounced in mice with small tumors. TGFa/c-myc mice (20-27 weeks old) with small (maximum diameter 1-3 mm, n=13, **f),** medium (3-7 mm, n=18, **g),** or large liver tumors (>7 mm, n=17, **h),** as graded by contrast-enhanced MRI, were tested. Blood was drawn 5 min before and 90 min after i. v. injection of iRGD, and serum AFP levels were determined by ELISA. AFP blood levels upon injection of PBS in TGFa/c-myc mice with HCC is shown for comparison **(i);** n. s., not significant; Wilcoxon signed rank test.
**Fig. 4****:** iRGD induces an increase in autotaxin (ATX) levels in HepG2-xenografted mice, and of PSA in LNCaP-xenografted mice.
   **a:** Blood was drawn from HepG2 xenografted nude mice (tumors 0.6-1.5 cm in diameter) 5 min before and 90 min after the i. v. injection of iRGD. The sera were analyzed for autotaxin (ATX) content (n=4 per group). Columns and error bars represent means ± SD.
   **b-d:** Blood was drawn from mice xenografted with LNCaP prostate cancers (tumors 0.8-1.3 cm in diameter) 5 min before and 90 min after the i. v. injection of iRGD (n=11) **(b)** or control peptide (n=5) **(c)** and analyzed for PSA content.
   **d:** Mean values ± SD of PSA levels following injection of the peptides. Asterisks indicate significant differences (*P<0.05); n. s., not significant.
   **a, d** Unpaired t-test; **b, c** Wilcoxon signed rank test.

### Materials and Methods:

Peptides. iRGD (CRGDKGPDC (SEQ ID No. 1)) and RGD control peptide (CRGDDGPKC (SEQ ID No. 5)) were synthesized by GenScript ([Piscataway, NJ], USA) as cyclic peptides with a disulfide bond between amino acids 1 and 9. The reported purity was higher than 98%.

Nude mouse experiments. All animal experiments have been approved by the local Ethics Animal Review Broad Darmstadt, Germany (F34/10, FK/1100). HepG2 and Huh-7 cells were obtained from ATCC (Manassas, VA) and RIKEN BioResource Center (Ibaraki, Japan), respectively, and were grown in DMEM supplemented with 10% FBS and penicillin/streptomycin (Life Technologies, Waltham, MA). LNCaP cells were obtained from the Leibnitz Institute DSMZ (Braunschweig, Germany). All cell lines were routinely monitored for morphologic and growth characteristics and mycoplasma. A total of 1-10×10⁶ cells (suspended in 100 ml of PBS) were injected subcutaneously into the flanks of NMRI Foxn1 nude mice (Envigo, Huntingdon, UK).

Mouse models of liver fibrosis. An autoimmune model of liver fibrosis was generated by injecting 6 weeks old FVB/NHsd mice (Envigo) with 2 × 108 pfu of Ad-2D6 i.p and i.v.30 Virus titers were determined with the Adeno-X rapid titer kit (Clontech, Palo Alto, CA). The mice were used for the experiments four weeks after the Ad-3D6 injection. For carbon tetrachloride (CCl₄)-induced liver fibrosis, FVB mice aged 6-8 weeks were treated twice weekly by intraperitoneal injection of 5 ml CCl₄ diluted 1:20 in corn oil for four weeks (31). The mice were used for the experiments on the day following the last CCl₄ injection.
As a third model of liver fibrosis, six months old Mdr2-/- mice in FBV background were used (32).

Generation of transgenic mice and visualization of HCC. The animals were inspected every 2-3 days. Male TGFa/c-myc mice were generated by crossing homozygous metallothionein/TGFα and albumin/c-myc mice in CD13B6CBA background as described (20, 21). After weaning, hepatocarcinogenesis in the mice was accelerated by ZnCl₂ in the drinking water. The endogenously formed HCCs were detected and monitored by gadoxetic acid (Gd-EOB-DTPA)-enhanced MRI in a 3T MRI scanner (Siemens Magnetom Trio, Siemens Medical Solution Health Service, Eschborn, Germany) as described recently (19, 33).

Treatment of the mice with peptides and use of anti-NRP-1 blocking antibody. The different mice received iRGD, RGD control peptide (4 µmol/kg each), or PBS by tail vein injection. Blood was drawn from the mice 5 min before and 90 min after the i. v. injection of peptides/vehicle, if not stated otherwise. AFP levels were determined in the blood samples.

For neutralization of NRP-1 in the HCC mice, TGF/c-myc mice with HCC according to Gd-EOB-DTPA-enhanced MRI and robust iRGD-induced increase in the blood AFP concentration one week before were injected intravenously with 50 µg of a neutralizing anti-mouse NRP-1 (R&D Systems, Minneapolis, MN). One hour later blood was drawn, followed by the injection of iRGD (4 µmol/kg) and a second blood draw after another 90 min. AFP levels were determined in sera from blood samples.

Hematoxylin and Eosin staining. Paraffin embedded liver sections were deparaffinized and rehydrated before incubating them in a hematoxylin bath for 8 min. The sections were washed in warm tap water for 10 min, rinsed in deionized water and 95% ethanol and counterstained in Eosin G/Y solution for 60 s. The sections were then dehydrated in 95% ethanol and pure ethanol, cleared in xylene and mounted.

Sirius Red staining. Paraffin embedded liver sections were deparaffinized and rehydrated before incubating the tissue with 100 ml Sirius Red solution (Electron Microscopy Science, Hatfield, PA) at room temperature for 1 h. Afterwards, slides were washed with 0.01 N HCl and H₂O, dehydrated ethanol, cleared in xylene and mounted.

ELISAs, serum ALT, AST and LDH measurements. To quantify AFP, total PSA and autotaxin, 20-50 µl of blood was collected 5 min before peptide injections. Sera were prepared and stored at - 20°C until utilization for the assays. Quantikine ELISAs to detect human autotaxin (DENP20, R&D Systems, Minneapolis, MN)34 as well as either mouse or human AFP were from R&D Systems (MAFP00 and DAFP00). The levels of total PSA were measured using the Elecsys total PSA immunoassay (Roche Diagnostics, Mannheim, Germany). There was no background from the mouse sera in these assays specific for the human substrates.
Alanine aminotransferase (ALT), aspartate aminotransferase (AST) and lactate dehydrogenase (LDH) levels in sera were measured by the Central Laboratory of the University Hospital Frankfurt.

Statistical analysis. The statistical significance of normalized blood levels of AFP, autotaxin and PSA were calculated by the Wilcoxon signed rank test. Comparisons between different treatment groups were performed by the unpaired, two-tailed t test (GraphPad Prism 8.1.2, GraphPad Software, San Diego, CA). Statistical significance was considered for p<0.05.

### Literature

1. Altekruse, S. F., Mcglynn, K. A., Dickie, L. A. & Kleiner, D. E. Hepatocellular carcinoma confirmation, treatment, and survival in surveillance, epidemiology, and end results registries, 1992-2008. Hepatology 55, 476-482 (2012).
2. Wang, J. H., Wang, C. C., Hung, C. H., Chen, C. L. & Lu, S. N. Survival comparison between surgical resection and radiofrequency ablation for patients in BCLC very early/early stage hepatocellular carcinoma. J. Hepatol. 56, 412-418 (2012).
3. Bray, F. et al. Global cancer statistics 2018: GLOBOCAN estimates of incidence and mortality worldwide for 36 cancers in 185 countries. CA Cancer J. Clin. 68, 394-424 (2018)
4. Abelev, G. I. Production of embryonal serum alpha-globulin by hepatomas: review of experimental and clinical data. Cancer Res. 28, 1344-1350 (1968).
5. Ruoslahti, E., Salaspuro, M., Pihko, H., Andersson, L. & Seppala, M. Serum alpha-fetoprotein: diagnostic significance in liver disease. Br. Med. J. 393, 527-529 (1974).
6. Catalona, W. J. et al. Measurement of prostate-specific antigen in serum as a screening test for prostate cancer. N. Engl. J. Med. 324, 1156-1161 (1991).
7. Borrebaeck, C. A. Precision diagnostics: moving towards protein biomarker signatures of clinical utility in cancer. Nat. Rev. Cancer 17, 199-204 (2017).
8. Teesalu, T., Sugahara, K. N., Kotamraju, V. R. & Ruoslahti, E. C-end rule peptides mediate neuropilin-1-dependent cell, vascular, and tissue penetration. Proc. Natl. Acad. Sci. USA 106, 16157-16162 (2009).
9. Sugahara, K. N. et al. Tissue-penetrating delivery of compounds and nanoparticles into tumors. Cancer Cell 16, 510-520 (2009).
10. Sugahara, K. N. et al. Coadministration of a tumor-penetrating peptide enhances the efficacy of cancer drugs. Science 328, 1031-1035 (2010).
11. Ruoslahti, E. Tumor penetrating peptides for improved drug delivery. Adv. Drug Deliv. Rev. 110-111, 3-12 (2017).
12. Pang, H. B. et al. An endocytosis pathway initiated through neuropilin-1, regulated by nutrient availability. Nat. Commun. 5, 4904- 4904 (2014).
13. Liu, X. et al. Tumor-penetrating peptide enhances transcytosis of silicasome-based chemotherapy for pancreatic cancer. J. Clin. Invest. 127, 2007-2018 (2017).
14. Feng, D., Nagy, J. A., Hipp, J., Dvorak, H. F. & Dvorak, A. M. Vesiculo-vacuolar organelles and the regulation of venule permeability to macromolecules by vascular permeability factor, histamine, and serotonin. J. Exp. Med. 183, 1981-1986 (1996) .
15. Ding, N. et al. iRGD synergizes with PD-1 knockout immunotherapy by enhancing lymphocyte infiltration in gastric cancer. Nat. Commun. 10, 1336 (2019).
16. Sugahara, K. N. et al. Tumor-penetrating iRGD peptide inhibits metastasis. Mol. Cancer Ther. 14, 120-128 (2015).
17. Pang, H. B. et al. A free cysteine prolongs the half-life of a homing peptide and improves its tumor-penetrating activity. J. Control. Release 175, 48-53 (2014).
18. Debruyne, E. N. & Delanghe, J. R. Diagnosing and monitoring hepatocellular carcinoma with alpha-fetoprotein: new aspects and applications. Clin. Chim. Acta 395, 19-26 (2008) .
19. Schmithals, C. et al. Improving drug penetrability with iRGD leverages the therapeutic response to sorafenib and doxorubicin in hepatocellular carcinoma. Cancer Res. 75, 3147-3154 (2015).
20. Murakami, H. et al. Transgenic mouse model for synergistic effects of nuclear oncogenes and growth factors in tumorigenesis: interaction of c-myc and transforming growth factor alpha in hepatic oncogenesis. Cancer Res. 53, 1719-1723 (1993) .
21. Haupenthal, J. et al. Reduced efficacy of the Plk1 inhibitor BI 2536 on the progression of hepatocellular carcinoma due to low intratumoral drug levels. Neoplasia 14, 410-419 (2012).
23. Gupta, S., Bent, S. & Kohlwes, J. Test characteristics of alpha-fetoprotein for detecting hepatocellular carcinoma in patients with hepatitis C. A systematic review and critical analysis. Ann. Intern. Med. 139, 46-50 (2003).
23. EASL Clinical Practice Guidelines: Management of hepatocellular carcinoma. J. Hepatol. 69, 182-236 (2018).
24. Moolenaar, W. H. & Perrakis, A. Insights into autotaxin: how to produce and present a lipid mediator. Nat. Rev. Mol. Cell Biol. 12, 674-679 (2011).
25. Desgrosellier, J. S. & Cheresh, D. A. Integrins in cancer: biological implications and therapeutic opportunities. Nat. Rev. Cancer 10, 9-22 (2010).
26. Pellet-Many, C., Frankel, P., Jia, H. & Zachary, I. Neuropilins: structure, function and role in disease. Biochem. J. 411, 211-226 (2008).
27. Nejjari, M. et al. Expression, regulation, and function of alpha V integrins in hepatocellular carcinoma: an in vivo and in vitro study. Hepatology 36, 418-426 (2002).
28. Berge, M. et al. Neuropilin-1 is upregulated in hepatocellular carcinoma and contributes to tumour growth and vascular remodelling. J. Hepatol. 55, 866-875 (2011).
29. Sugahara, K. N. et al. A tumor-penetrating peptide enhances circulation-independent targeting of peritoneal carcinomatosis. J. Control. Release 212, 59-69 (2015).
30. Holdener, M. et al. Breaking tolerance to the natural human liver autoantigen cytochrome P450 2D6 by virus infection. J. Exp. Med. 205, 1409-1422 (2008).
31. Hintermann, E., Bayer, M., Pfeilschifter, J. M., Luster, A. D. & Christen, U. CXCL10 promotes liver fibrosis by prevention of NK cell mediated hepatic stellate cell inactivation. J. Autoimmun. 35, 424-435 (2010).
32. Hintermann, E. et al. Junctional adhesion molecules JAM-B and JAM-C promote autoimmune-mediated liver fibrosis in mice. J. Autoimmun. 91, 83-96 (2018).
33. Ibrahim, A. A. et al. Hypoxia causes downregulation of Dicer in hepatocellular carcinoma, which is required for upregulation of hypoxia-inducible factor 1α and epithelial-mesenchymal transition. Clin. Cancer Res. 23, 3896-3905 (2017).
34. Pleli, T. et al. Serum autotaxin is a parameter for the severity of liver cirrhosis and overall survival in patients with liver cirrhosis - a prospective cohort study. PLoS ONE 9, e103532 (2014).
35. Moolenaar, W. H. & Perrakis, A. Insights into autotaxin: how to produce and present a lipid mediator. Nat. Rev. Mol. Cell Biol. 12, 674-679 (2011).

## Claims

1. A method for the diagnosis and/or risk stratification of carcinoma diseases, **characterized in that** by the following steps
a.) iRGD is applied to a subject,
b.) a sample of a bodily fluid is provided from said subject,
c.) determining or identifying in said sample one or more marker released from a tumor into the bodily fluid.

2. The method for the diagnosis and/or risk stratification of carcinoma diseases according to claim 1, wherein in step c.) the concentration or level of a marker is determined and d.) correlating the determined concentration or level to a potential risk for carcinoma.

3. The method for the diagnosis and/or risk stratification of carcinoma diseases according to claim 1 or claim 2, wherein step c.) correlates to i.) an altered, particularly increased concentration or level of a marker indicating a tumor or a tumor activity or ii.) an identified marker indicating the presence of a tumor.

4. The method for the diagnosis and/or risk stratification of carcinoma diseases according to any one of the preceding claims, **characterized in that** iRGD is administered via intravenous, intramuscular or subcutaneous application.

5. The method for the diagnosis and/or risk stratification of carcinoma diseases according to any one of the preceding claims, **characterized in that** the concentration or level of at least one marker is determined before and after the administration of iRGD, in particular independent from each other one week, preferably three days, 48 hours or 24 hours.

6. The method for the diagnosis and/or risk stratification of carcinoma diseases according to any one of the preceding claims, comprising the additional steps a'.) and c'.), wherein
a'.) a first sample of a bodily fluid is provided from a subject,
a.) iRGD is applied to said subject,
b.) a second sample of a bodily fluid is provided from said subject,
c.) determining or identifying in said sample one or more marker released from a tumor into the bodily fluid and
c'.) determining the difference of the marker levels between the first sample a'.) and second sample b.).

7. The method for the diagnosis and/or risk stratification of carcinoma diseases according to any one of the preceding claims, wherein the released marker into the bodily fluid is selected from the group of tumor markers, like circulating tumor markers or tumor tissue markers or any chemical substances, preferably proteins, like autotaxin (ATX).

8. The method for the diagnosis and/or risk stratification of carcinoma diseases according to any one of the preceding claims, **characterized in that** at least one tumor marker is selected from the group consisting of carcinoembryonic antigen (CEA), alpha fetoprotein (AFP), carbohydrate antigen 19/9 (CA 19-9), cancer antigen 72/4 (CA 72-4), cancer antigen 125, cancer antigen 15/3 (CA 15-3), neuron-specific enolase (NSE), squamous cell carcinoma antigen (SCC), cytokeratin fragment (CYFRA), human chorionic gonadotropin (HCG), prostate-specific antigen (PSA), human thyroglobulin (HTG), mucin-like cancer associated antigen (MCA), nuclear matrix protein 22 (NMP22), human placental alkaline phosphatase (hPALP), calcitonin, myopodin, prostate-specific membrane antigen (PSMA), pyruvate kinase M2 (PKM2), S100 proteins, thymidine kinase (TK) and tissue polypeptide antigen (TPA).

9. The method for the diagnosis and/or risk stratification of carcinoma diseases according to any one of the preceding claims, **characterized in that** the carcinoma is selected from the group consisting of solid malignant neoplasms, breast cancer, colon cancer, lung cancer, pancreatic cancer, stomach cancer, ovarian cancer, biliary duct cancer, prostate cancer, cervical cancer, glioblastoma, bronchial cancer, renal cell cancer, bladder cancer, sarcomas, liver cancer, hepatocellular carcinoma (HCC), brain tumors, seminal vesicle tumors, thyroid cancer, medullary thyroid cancer, melanoma, leukemia, lymphoma or tumor metastases of these cancers.

10. The method for the diagnosis and/or risk stratification of carcinoma diseases according to any one of the preceding claims, **characterized in that** the iRGD is selected from the group having the sequences CRGDKGPDC (SEQ ID No.1), CRGDRGPDC (SEQ ID No.2), CRGDKGPEC (SEQ ID No.3), CRGDRGPEC (SEQ ID No.4).

11. The method for the diagnosis and/or risk stratification according to any one of the preceding claims, for the identification of patients who are at an increased risk of and/or have an unfavorable prognosis for carcinoma diseases.

12. The method for the diagnosis and/or risk stratification according to any one of the preceding claims, wherein the patient is a symptomatic and/or asymptomatic patient.

13. The method for the diagnosis and/or risk stratification according to any one of the preceding claims, for the therapy control of carcinoma diseases.

14. The method for the diagnosis and/or risk stratification of carcinoma diseases according to any one of the preceding claims, for carrying out clinical decisions, in particular advanced treatments and therapies using drugs, including the decision to hospitalize the patient.

15. The method for the diagnosis and/or risk stratification of carcinoma diseases according to any one of the preceding claims, for the prognosis, for early detection and detection by differential diagnosis, for assessment of the severity, and for assessment of the course of the disease concomitant with the therapy.

16. Use of iRGD as a marker enhancer or booster in a method for the diagnosis and/or risk stratification of carcinoma diseases or carcinomas according to one of claims 1 to 15.

17. A kit comprising one or more injection solutions for carrying out a method according to any one of claims 1 to 15, comprising iRGD and means for detecting at least one tumor marker.


SEQUENCE LISTING

<110>  Piiper, Albrecht

<120>  Method for Diagnosing Carcinomas Using iRGD as a Tumor Marker
       Booster

<130>  API196-04EP

<140>  EP20175139.3
<141>  2020-05-18

<160>  4

<170>  PatentIn version 3.5

<210>  1
<211>  9
<212>  PRT
<213>  Artificial

<220>
<223>  Tumorhoming-Peptid

<400>  1

Cys Arg Gly Asp Lys Gly Pro Asp Cys
1               5


<210>  2
<211>  9
<212>  PRT
<213>  Artificial

<220>
<223>  Tumorhoming-Peptid

<400>  2

Cys Arg Gly Asp Arg Gly Pro Asp Cys
1               5


<210>  3
<211>  9
<212>  PRT
<213>  Artificial

<220>
<223>  Tumorhoming-Peptide

<400>  3

Cys Arg Gly Asp Lys Gly Pro Glu Cys
1               5


<210>  4
<211>  9
<212>  PRT
<213>  Artificial

<220>
<223>  Tumorhoming-Peptide

<400>  4

Cys Arg Gly Asp Arg Gly Pro Glu Cys
1               5